# EUROPEAN PATENT SPECIFICATION

(11) **EP 0 583 907 B1**
(45) Date of publication and mention of the grant of the patent: **26.03.1997**
(21) Application number: 93306079.0
(22) Date of filing: 02.08.1993
(51) Int. Cl.: C07D 323/06

(54) **Process for producing trioxan**
Verfahren zur Erzeugung von Trioxan
Procédé de production de trioxanne

(30) Priority: 04.08.1992 JP 208265/92
(43) Date of publication of application: 23.02.1994
(73) Proprietor: POLYPLASTICS CO. LTD., Chuo-Ku Osaka-shi Osaka 541 (JP)
(72) Inventor: Kashihara, Osamu, Shizuoka (JP); Kuroishi, Takeo, Shizuoka (JP); Hiragohri, Motohito, Shizuoka (JP); Akiyama, Minoru, Shizuoka (JP); Kasai, Yuzo, Shizuoka (JP); Fukui, Yuichi, Shizuoka (JP)
(74) Representative: Jackson, Peter

(56) References cited:
- EP-A- 0 187 261
- FR-A- 1 427 477
- FR-A- 2 123 298
- CHEMICAL ABSTRACTS, vol. 115, no. 15, 14 October 1991, Columbus, Ohio, US; abstract no. 159187f, page 947 ; & JP-A-03 145 485 (ASAHI CHEMICALL INDUSTRY CO LTD) 20 June 1991
- CHEMICAL ABSTRACTS, vol. 115, no. 15, 14 October 1991, Columbus, Ohio, US; abstract no. 159186e, page 947 ; & JP-A-03 123 777 (ASAHI CHEMICAL INDUSTY CO LTD) 27 May 1991

## Description

The present invention relates to a process for producing trioxane, which is the feedstock material for producing polyoxymethylene, from an aqueous solution of formaldehyde. More particularly, it provides a process for producing trioxane from an aqueous formaldehyde solution which aims at saving energy throughput the whole process.

In general, a process for producing trioxane from an aqueous solution of formaldehyde comprises the following steps:
(1) synthesizing trioxane from the aqueous solution of formaldehyde in the presence of a catalyst;
(2) separating the aqueous solution of formaldehyde containing the trioxane thus synthesized; and
(3) separating trioxane from the aqueous solution of formaldehyde containing trioxane, followed by the purification thereof.

One prior-art process for producing pure trioxane comprises synthesizing trioxane by heating and distilling an aqueous solution of formaldehyde of a concentration of 30 to 70% by weight in the presence of a liquid acid such as sulfuric acid and taking an aqueous solution of formaldehyde containing the trioxane thus synthesized out of the reaction system to obtain a distillate comprising from 20 to 55% by weight of trioxane, from 17 to 35% by weight of formaldehyde and from 20 to 50% by weight of water, extracting the distillate with a solvent insoluble or hardly soluble in water, and rectifying the extract to separate trioxane (Japanese Patent Publication-B No. 6344/1966).

There are disclosed other processes wherein the liquid acid is replaced by various solid acids as the synthesis catalyst in, for example, Japanese Patent Publication No. 12794/1965, Japanese Patent Publication-A No. 203985/1983 and Japanese Patent Publication-A No. 134789/1984.

Furthermore, there have been proposed various solvents to be used for extracting trioxane from the trioxane-containing aqueous solution of formaldehyde which is taken out of the reaction system by distilling or evaporating.

Further, Japanese Patent Publication-A No. 145485/1991 discloses a process for producing trioxane which comprises feeding an aqueous solution of formaldehyde of a concentration of from 30 to 85% by weight into a distillation column, pouring a liquid drawn from the bottom of the column into a reactor packed with an acid catalyst, in particular, a solid acid catalyst, and circulating a trioxane-containing aqueous solution of formaldehyde effusing from the reactor to the intermediate tray(s) of the distillation column.

However these prior art processes require much energy, since they comprise synthesizing trioxane from an aqueous solution of formaldehyde in the presence of an acid catalyst, taking a trioxane-containing aqueous solution of formaldehyde out of the reaction system and, if necessary, concentrating trioxane by distilling or evaporating.

When trioxane is taken out of the reaction system by distilling or evaporating, the vapor-liquid equilibrium among trioxane, formaldehyde and water makes it unavoidable that the gaseous phase (distillate) rich in trioxane also contains a large amount of water, as described in the above-mentioned Japanese Patent Publication-B No. 6344/1966. The evaporation of this water is accompanied by the loss of much heat of evaporation (sensible heat and latent heat), which increases the energy required for the production of trioxane.

Further, Japanese Patent Publication-A No. 49250/1992 proposes a process for extracting trioxane from a liquid reaction mixture by combining a solid acid catalyst reaction step with an extraction step. This process comprises preparing two or more reactors, synthesizing trioxane through a reaction under forced circulation of an aqueous solution of formaldehyde or a mixture of an aqueous solution of formaldehyde with an extractant in one or more reactors, contacting the reaction mixture with the extractant to thereby extract and separate trioxane, and using the aqueous formaldehyde solution, from which trioxane has been extracted, as the feedstock material for synthesizing trioxane in another reactor. In this process, at least two reactors are indispensable and the use of either extractors in a number corresponding to that of the reactors or a single extractor (column) of a complicated constitution is unavoidable, which costs a great deal for the construction of the reaction system. When the aqueous formaldehyde solution containing the trioxane thus synthesized is contacted with the extractant and the solid acid catalyst at the same time, there is a risk that a reaction might proceed among these substances, when employed for a prolonged period of time, and the catalyst is consequently degraded and the reaction yield lowered. In the process disclosed in the above-mentioned patent, trioxane is synthesized in the first reactor and extracted in the extractor and then the aqueous formaldehyde solution, the formaldehyde concentration of which has been lowered, is fed into another reactor to be used therein for synthesizing trioxane, said operations being repeated successively. According to this process, although the apparent conversion of the feedstock formaldehyde into trioxane is increased, the reaction equilibrium of trioxane is successively lowered in the second reactor and thereafter, since the synthesis of trioxane from formaldehyde is an equilibrium reaction. Thus the extraction efficiency is successively lowered and the subsequent isolation of trioxane becomes difficult. In particular, when trioxane is extracted with an organic solvent having a boiling point lower than that of trioxane, as described in the Example of the Japanese Patent Publication-A No. 49250/1992, it is anticipated that the decrease in the reaction equilibrium of trioxane and the decrease in the extraction efficiency accompanying the same would make it necessary to consume much energy in the subsequent operation for separating and purifying trioxane.

The conventional processes for producing trioxane as described above have not attempted to achieve significant reductions in energy consumption. Specifically there has been no technical proposal for significant energy savings throughout the whole process.

The present invention is based on the concept of saving energy in the production of pure trioxane from an aqueous solution of formaldehyde, by extracting trioxane from the synthesis and reaction system with an organic solvent but without causing any phase change of water and the organic solvent, and subsequently purifying the trioxane.

Accordingly, the present invention relates to a process for producing trioxane from an aqueous solution of formaldehyde, characterized by the following steps:
(1) preparing an aqueous solution of formaldehyde of a high concentration, the formaldehyde concentration of said solution being within a starting range of 40 to 85% by weight;
(2) transferring the aqueous solution of formaldehyde of a high concentration obtained in the above step (1) into a reactor packed with a solid acid catalyst to form trioxane;
(3) introducing the aqueous solution of formaldehyde containing the trioxane produced in step (2) into an extractor without causing any phase change to extract the trioxane with a water-insoluble organic solvent having a boiling point higher than that of trioxane and being free from any azeotropic composition with trioxane; and
(4) elevating, after the completion of the extraction of the above step (3), the formaldehyde concentration of the aqueous phase and returning the aqueous phase to the reactor of the step (2), while separating the organic solvent phase into trioxane and the organic solvent by distillation and taking trioxane out of the top of a distillation column and returning the organic solvent obtained from the bottom of the distillation column to the step (3).

The process according to the present invention is characterized in various aspects. Firstly, when trioxane is synthesized in an aqueous formaldehyde solution and the thus formed trioxane-containing aqueous solution is taken out of the reaction system, there is no need to concentrate the trioxane by boiling the aqueous solution, unlike the conventional processes, for example, those described in the Japanese Patent Publication-B No. 6344/1966 and Japanese Patent Publication-A No. 145485/1991. Namely, the process of the present invention, wherein the synthesized trioxane is separated from the feedstock formaldehyde solution without causing any phase change of water, does not require as much energy as the prior art in the evaporation of water. Secondly, the process of the present invention does not necessitate such a complicated procedure as the one employed in the process described in the Japanese Patent Publication-A No. 49250/1992 and the trioxane equilibrium concentration in the reaction mixture can be maintained at a high level, since an aqueous formaldehyde solution of a concentration continuously regulated to a high level can be fed into the reactor. Thus the extraction of trioxane from the reaction mixture and the subsequent separation and purification of the same can be efficiently conducted. Thirdly, the process of the present invention is characterized in that the extractant is selected by considering the rationalization of the procedure for separating and purifying the extracted trioxane, thus providing a process for producing trioxane, whereby significant energy saving can be realized by improving the efficiency throughout the whole process of the synthesis of trioxane from an aqueous formaldehyde solution to the purification of trioxane.

The feedstock aqueous solution to be introduced into the synthesis reactor [i.e. for step (2)] should have a high concentration of formaldehyde. In general, the concentration of the formaldehyde solution is adjusted to 40 to 85% by weight (based on formaldehyde) in this step. If the concentration of the formaldehyde solution is less than 40% by weight, only a poor conversion of formaldehyde into trioxane is achieved. If however this concentration exceeds 85% by weight, the formation of paraformaldehyde makes the operation difficult. It is preferable to adjust the formaldehyde concentration to 55 to 80% by weight, still preferably 60 to 75% by weight.

The above-mentioned aqueous formaldehyde solution of a high concentration may be prepared from an aqueous formaldehyde solution or a formaldehyde gas obtained by common methods for producing formaldehyde, for example, the methanol oxidation method (the so-called excess air method and the excess methanol method) and the methylal method (refer to, for example, Japanese Patent Publication-B No. 287051/1989). These starting materials may be concentrated or diluted by operations commonly employed in the art, for example, absorption, evaporation or distillation. More precisely, when the feedstock aqueous formaldehyde solution has a formaldehyde concentration less than the desired level, it may be concentrated by evaporation or distillation to adjust it to the desired concentration. If the starting material is an aqueous formaldehyde solution having a formaldehyde concentration exceeding the desired level or a formaldehyde gas, then water or an aqueous formaldehyde solution may be added to adjust it to the desired concentration.

The aqueous solution of formaldehyde of a high concentration is transferred to a reactor packed with a solid acid catalyst to form trioxane [step (2)].

The solid acid catalyst may be any of an organic solid acid, an inorganic solid acid and a mixture comprising two or more of them. Examples of the organic solid acid include ion-exchange resins having sulfonate or fluoroalkanesulfonate groups and examples of the inorganic solid acid include inorganic oxide composites, such as acid clay, silica, alumina, silica/alumina, alumina/boria and zeolite, and acid carriers impregnated with, for example, sulfuric, phosphoric or boric acid. The type of the body of the reactor in which these solid acid catalysts are to be packed may be arbitrarily selected from among, for example, those of packed column, pipe, basket, fluidized bed and plate column types. Factors relating to the reaction, such as the type of the catalyst, the amount of the catalyst packed in the reactor, the feed rate of the aqueous formaldehyde solution, the residence time and the reaction temperature, cannot be determined indiscriminately, because they relate to each other. Thus, these factors are to be determined in such a manner that the conversion from formaldehyde to trioxane proceeds smoothly in the reactor and, in general, the trioxane concentration at the outlet of the reactor is substantially in equilibrium with that of the feedstock aqueous formaldehyde solution. In the determination of these factors, the capacity of the reactor may be determined while taking the reactivity and the heat resistance of the catalyst, the output and the practical usefulness into consideration. Among these factors, the reaction temperature may preferably range from 85 to 130°C in general. It is also preferable to set other conditions in such a manner as to give a residence time in the reactor of from 25 seconds to 25 minutes in usual. If the residence time is shorter than 25 seconds, the reaction cannot fully proceed even when a highly reactive catalyst is selected and the reaction is difficult to control. If however the residence time exceeds 25 minutes, a side reaction, i.e., Cannizzaro's reaction is promoted. In this case, the disadvantage may arise of having to use a large reactor.

In the step of contacting the aqueous solution of formaldehyde and trioxane with an organic solvent [step (3)], the said aqueous is introduced into an extractor without causing any phase change and the trioxane is extracted with a water-insoluble organic solvent which has a boiling point higher than that of trioxane and is free from any azeotropic composition with trioxane. It is preferable to use an organic solvent having a density of from 0.8 to 0.95 or from 1.05 to 1.15 and giving a partition ratio of trioxane in the organic solvent to the aqueous formaldehyde solution of 0.5 or above.

A solvent having a boiling point higher than that of trioxane is used because it is advantageous from the viewpoint of the energy balance to distill the trioxane, which is the minor component, into the column top side (gaseous phase) in the distillation after the extraction. It is necessary to use an organic solvent free from any azeotropic composition with trioxane in order not to complicate the operation after the distillation. Further, the density of the organic solvent is preferably controlled to be within the range as specified above in order to facilitate the liquid dispersion and phase dispersion in the extraction. Furthermore, the partition ratio is preferably regulated to 0.5 or above in order to secure an extraction efficiency above a certain level.

Suitable examples of the organic solvent satisfying the above-mentioned requirements include saturated aliphatic hydrocarbons, halogenated aliphatic hydrocarbons, alicyclic hydrocarbons, aromatic hydrocarbons, alkylated aromatics and halogenated aromatic compounds. More particularly, preferable examples thereof include nonane, 1-chlorooctane, naphthalene, phenyl ether, anisole, monochlorobenzene, dichlorobenzenes such as o-chlorobenzene, xylenes, acetophenone, benzophenone, diethylbenzene, cumene, mesitylene and biphenyl.

The method for extracting trioxane in the step (3) is not particularly restricted, and may employ an extractor as commonly employed in the art. For example, a packed column or a tray column may be used therefor and the contact may be effected either by the counter flow method or by the parallel flow method. After the completion of the extraction, the composition of the trioxane contained in the organic solvent usually depends on the reaction equilibrium concentration depending on the concentration of the aqueous formaldehyde solution in the synthesis of trioxane and the partition of trioxane in the organic solvent. It usually ranges from 1 to 10% by weight.

The present invention is characterized by combining the procedure, whereby the trioxane-containing aqueous formaldehyde solution thus obtained in the reactor is introduced into an extractor without causing any phase change to extract the trioxane with a solvent, with the use of an extractant satisfying the above-mentioned requirements, thus largely contributing to the production of pure trioxane by using less energy. Such an effect is difficult to achieve if trioxane is separated from the reaction system by distilling or evaporating or if trioxane is extracted with a solvent which does not satisfy the above-mentioned requirements.

After the completion of the extraction of trioxane from the aqueous phase [step (3)], the formaldehyde concentration of the aqueous phase is elevated and then the aqueous phase is returned to the synthesis reactor [for step (2)], while the organic phase is further separated into trioxane and the organic solvent by distillation and the trioxane is recovered as the distillate, from the top of the distillation column. The organic solvent is obtained from the bottom of the distillation column and is recycled for re-use [in step (3)].

The aqueous phase to be returned to the synthesis reactor has its formaldehyde concentration increased by the concentration procedure of the step (1) or by using, for example, a concentrator which is separately provided. If more than one reactors are used, the aqueous formaldehyde solution may be partitioned at an arbitrary ratio and then returned to each reactor. Alternately, it may be selectively returned to a certain reactor. On the other hand, the organic solvent phase is separated into trioxane and the organic solvent by distillation. Thus trioxane usable in polymerization is obtained from the top of the distillation column while the organic solvent obtained from the bottom of the distillation column is recycled.

The distillation columns to be used in the process of the invention include a tray column, a bubble-cap column and a packed column. Various distillation methods are usable depending on the organic solvent to be used and the concentration of trioxane contained in the solution. If the extractant contains trace amounts of moisture or low-boiling materials such as side products dissolved therein, it is preferable to effect distillation for eliminating these materials to the distillation for obtaining trioxane.

The process of the present invention for producing trioxane makes it possible to achieve energy saving throughout the whole process for producing trioxane from an aqueous solution of formaldehyde, with consequential savings in production costs.

A further advantage of the process of the present invention is that the organic solvent to be used in the extraction of trioxane does not flow into the synthesis reactor. Thus the degradation of the catalyst otherwise caused by the organic extractant is prevented and, as a result, a high reaction ratio can be maintained for a prolonged period of time.

Furthermore, the process of the present invention can be easily effected by a less complicated system than those employed in the conventional processes.

The invention is further illustrated with reference to the accompanying drawings, in which:
Fig. 1 is a flow diagram showing one example of an embodiment of the process of the present invention for producing trioxane.
Fig. 2 is a flow diagram showing an embodiment of a conventional process for producing trioxane.
Fig. 3 is a flow diagram showing another example of the process flow.

In the said Figures, the reference numerals indicate the following components:
1: still pot,
2: reactor,
3: extraction column,
4: distillation column, and
5: pre-extraction distillation column.

### Examples

The following non-limiting Examples, which include a description of the various flow diagrams shown in the Figures, further illustrate the present invention

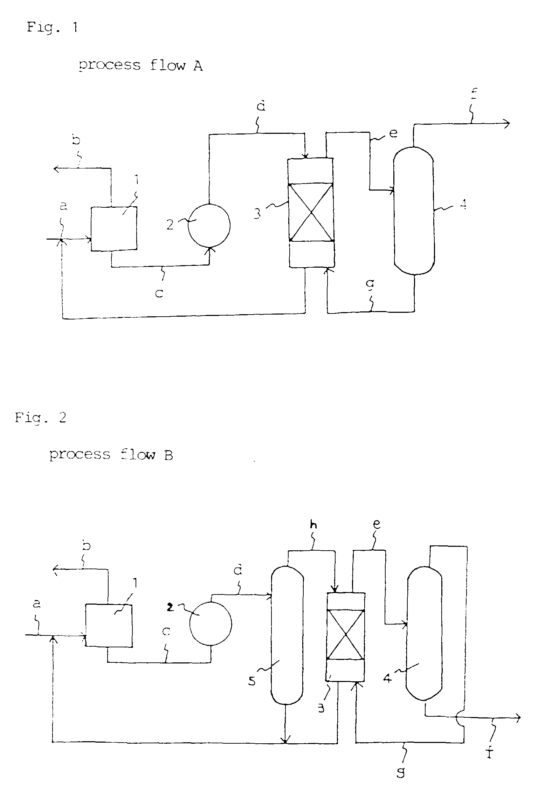

In the following Examples 1 to 5, procedures are carried out in accordance with the process flow A of Fig. 1.

### Example 1

An experimental system was constructed in accordance with the process flow A of Fig. 1. In Fig. 1, numeral 1 refers to a still pot of a capacity of 3 l, numeral 2 to a reactor filled with 0.2 l (200 cm³) of Nafion NR50 (a fluororesin ion-exchange resin manufactured by du Pont), numeral 3 to a packed extraction column (inner diameter: 300 mm, height: 1.5 m) filled with Raschig rings and numeral 4 to a distillation column (inner diameter: 300 mm) having 40 sieve trays therein.

First, a 50% by weight aqueous solution of formaldehyde was fed from the line a at a rate of 100 g/hr and concentrated in the still pot 1 in such a manner as to give a 60% by weight aqueous solution of formaldehyde including the one recovered from the extraction column 3 [step (1)]. Next, the concentrated aqueous formaldehyde solution was transferred to the reactor 2 maintained at 95°C via the line c to obtain an aqueous formaldehyde solution containing 3.5% by weight of trioxane [step (2)]. Next, the solution thus obtained was introduced into the extraction column 3 via the line d while feeding o-dichlorobenzene into the same column 3 from the line g at a flow rate of 2,800 ml/hr to extract trioxane from the aqueous formaldehyde solution [step (3)]. In practice, dichlorobenzene, which had a high specific gravity, was fed from the column top and the aqueous formaldehyde solution containing trioxane was fed from the lower part of the column, while the extract containing trioxane was drawn from the column bottom. Next, the extract thus obtained was fed into the distillation column 4 via the line e for distillation. Thus, 43 g/hr of trioxane was obtained from the top of the distillation column 4 via the line f [step (4)].

Steam was applied mostly to a heater for distillation. A small amount of steam was applied to heaters for the reactor and transporting pipes.

In this Example, a total of 1.77 g of steam was required in order to obtain 1 g of trioxane and no o-dichlorobenzene flowed into the reactor 2.

### Example 2

The procedure of the above Example 1 was repeated except that 0.3 l (300 cm³) of Diaion SK108 (strongly acidic styrenic cation-exchange resin manufactured by Mitsubishi Chemical Industries, Ltd.) was used as the catalyst to be packed in the reactor 2, that mesitylene was employed as the organic extractant and that it was fed from the line g at a flow rate of 15,000 ml/hr.

In this Example, 1.77 g of steam was required in order to obtain 1 g of trioxane and no solvent flowed into the reactor 2.

### Examples 3 to 5

The procedure of the Example 1 was repeated except that the catalyst, the organic extractant and the flow rate of the organic extractant from the line g were altered each as specified in Table 1, similar to Example 2. Table 1 shows the results of Examples 3 to 5 together with those of Examples 1 and 2.

### Comparative Example 1

Fig. 2 shows one example of a process flow for the embodiment of a conventional method for producing trioxane. In this Example, an experimental system was constructed in accordance with the process flow B of Fig. 2 and the procedure was performed.

In Fig. 2, numeral 1 refers to a still pot of a capacity of 3 l, numeral 2 to a heat evaporation type reactor of a capacity of 5 l to which 2% by weight of sulfuric acid was fed, numeral 5 to a distillation column (inner diameter: 300 mm) having 20 sieve trays therein, numeral 3 to a packed extraction column (inner diameter: 300 mm, height: 0.5 m) filled with Raschig rings, and numeral 4 to a distillation column (inner diameter: 300 mm) having 20 sieve trays therein.

Similar to the Example 1, a 50% by weight aqueous solution of formaldehyde was first fed from the line a at a rate of 100 g/hr and concentrated in the still pot 1 in such a manner as to give a 60% by weight aqueous solution of formaldehyde including the recovered one. Next, the concentrated aqueous formaldehyde solution was transferred to the reactor 2 via the line c and then evaporated therein under heating. Thus an aqueous formaldehyde solution containing 17% by weight of trioxane was obtained from the line d. Next, this aqueous solution was further distilled in the distillation column 5 to obtain an aqueous formaldehyde solution containing 41% by solution was weight of trioxane from the column top. This then introduced into the extraction column 3 via the line h column 3 from the line y while feeding benzene into the same from the at a flow rate of 200 ml/hr to extract trioxane the obtained extract was aqueous formaldehyde solution. Then the line e and fed into the distillation column 4 via the distilled therein. 40 g/hr of trioxane was obtained from bottom of this distillation column 4.

In this Comparative Example, 9.00 g of steam was required in order to obtain 1 g of trioxane.

### Comparative Example 2

A system of the same construction as the one employed in the Comparative Example 1 was prepared and the same procedure was performed except that a nonevaporation type reactor filled with 250 ml of Diaion pk216 was employed as the reactor 2 and that a distillation column of a inner diameter of 300 mm having 40 sieve trays therein was employed as the distillation column 5. Thus an aqueous formaldehyde solution containing 3.5% by weight of trioxane was obtained in the line d. Subsequently, the procedure of the Comparative Example 1 was repeated. Table I shows the result.

### Comparative Example 3

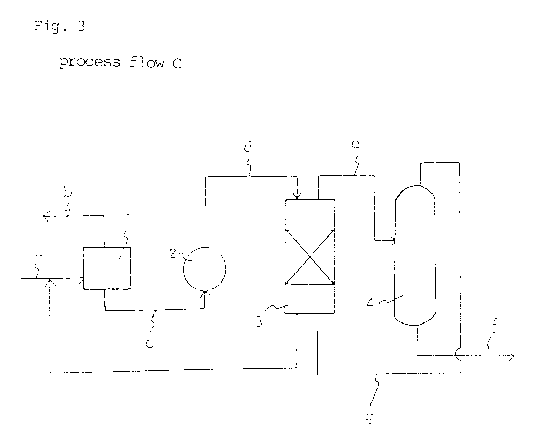

Fig. 3 shows another example of a process flow. In this Example, an experimental system was constructed in accordance with the process flow C of the Fig. 3. The construction of this system in accordance with the process flow C was the same as that employed in the Example 1 which was constructed in accordance with the process flow A, except that a reactor filled with 250 ml of Diaion pk216 was employed as a reactor corresponding to the reactor 2 in the process flow A, that a solvent having a boiling point lower than that of trioxane was employed as an extractant, and that the trioxane distilled in the distillation column 4 was drawn from the bottom of the distillation column 4 via the line f.

In this Example, trioxane was produced by the same method as the one described in the Example 1 except that benzene was used as the extractant and fed into the extraction column 3 via the line g at a flow rate of 1,500 ml/hr. As a result, the concentration of the trioxane extracted into the benzene phase was low and, furthermore, an azeotropic composition of benzene with trioxane was formed during the distillation in the distillation column 4. When it was attempted to recover trioxane from the column bottom, it was therefore unavoidable that the trioxane was contaminated with a large amount of benzene. When it was attempted to distill off the whole benzene from the column top, it became impossible to effect distillation and separation.

### Comparative Example 4

Trioxane was produced by the same method as described in the Example 3, except that ethylbenzene was used as the organic extractant. Although ethylbenzene was a solvent having a boiling point higher than that of trioxane, it formed an azeotropic composition with trioxane. In the distillation, therefore, an azeotrope of trioxane with ethylbenzene distilled from the column top, thus making isolation of trioxane impossible.

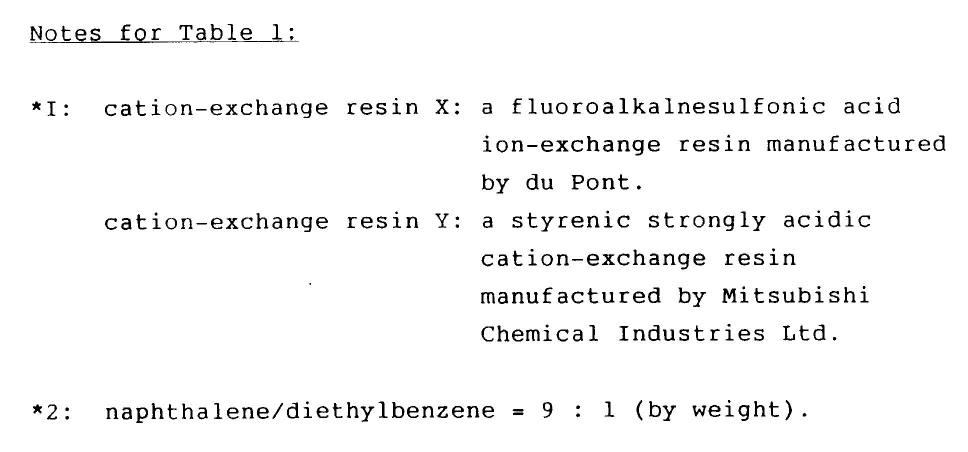

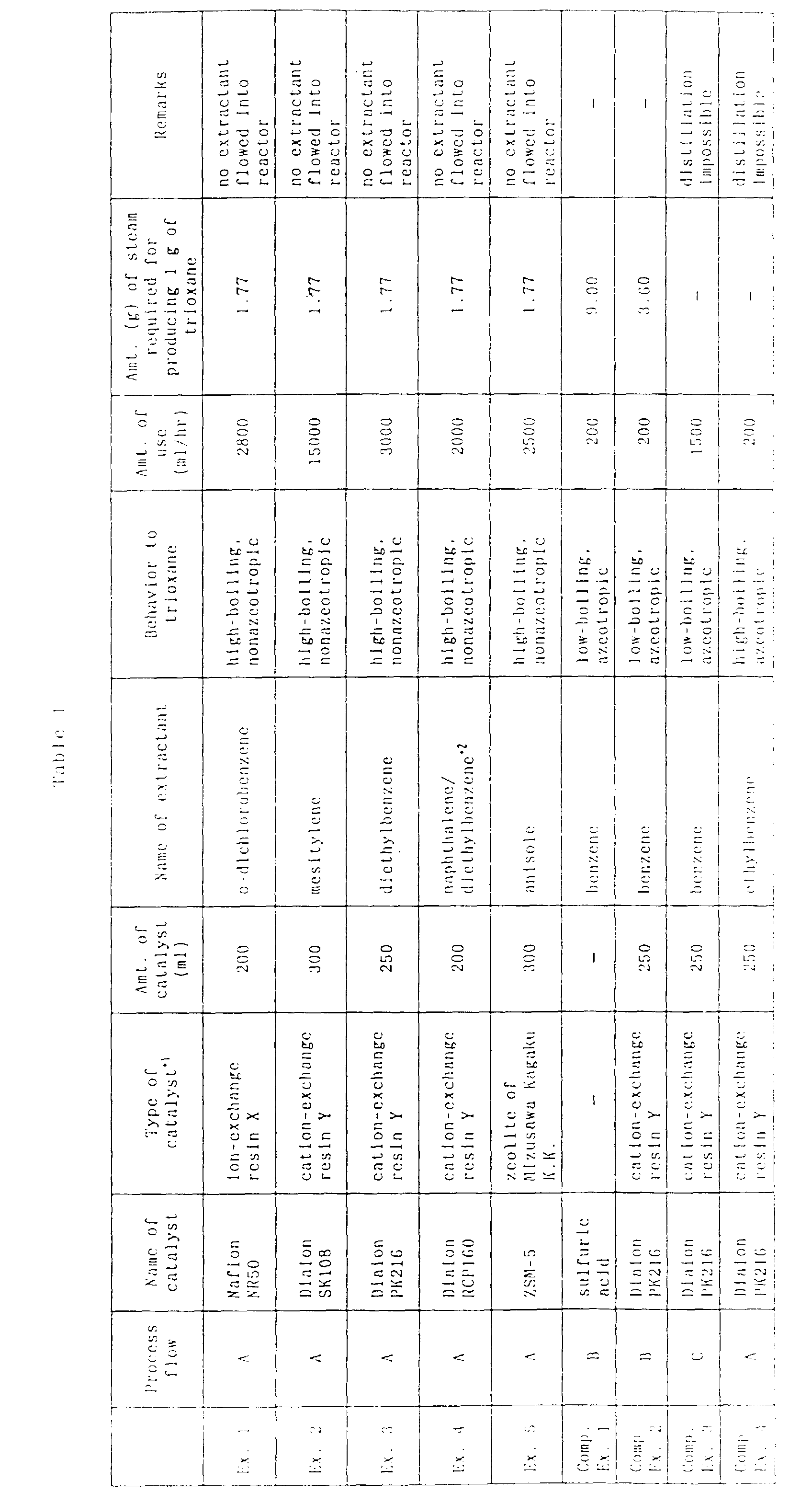

## Claims

1. A process for producing trioxane from an aqueous solution of formaldehyde, characterized by comprising the following steps (1) to (4):
(1) preparing an aqueous solution of formaldehyde of a high concentration, the formaldehyde concentration of said solution being within a starting range of 40 to 85% by weight;
(2) transferring the aqueous solution of formaldehyde of a high concentration obtained in the above step (1) into a reactor packed with a solid acid catalyst to thereby form trioxane;
(3) introducing the aqueous solution of formaldehyde containing the trioxane produced in the above step (2) into an extractor without causing any phase change to extract the trioxane with a water-insoluble organic solvent having a boiling point higher than that of trioxane and being free from any azeotropic composition with trioxane; and
(4) elevating, after the completion of the extraction of the above step (3), the formaldehyde concentration of the aqueous phase and returning the aqueous phase to the reactor of the step (2), while separating the organic solvent phase into trioxane and the organic solvent by distillation and taking trioxane out of the top of a distillation column and returning the organic solvent obtained from the bottom of the distillation column to the step (3).

2. A process as claimed in claim 1, wherein the starting range of the formaldehyde concentration of the aqueous solution of formaldehyde to be used for the trioxane synthesis reaction is from 55 to 80% by weight.

3. A process as claimed in claim 2, wherein the starting range of the formaldehyde concentration of the aqueous solution of formaldehyde to be used for the trioxane synthesis reaction is from 60 to 75% by weight.

4. A process as claimed in any preceding claim, wherein the said aqueous solution is obtained either by concentrating, when the aqueous formaldehyde solution has a formaldehyde concentration less than 40% by weight, said solution by evaporating or distilling to the desired concentration, or by adding, when the aqueous formaldehyde solution has a formaldehyde concentration exceeding 85% by weight or a formaldehyde gas is used as the feedstock material, water or an aqueous formaldehyde solution to the feedstock material to give the desired concentration.

5. A process as claimed in any preceding claim, wherein the solid acid catalyst comprises an inorganic solid acid, an organic solid acid or a mixture thereof.

6. A process as claimed in any preceding claim, wherein the organic solvent has a density of from 0.8 to 0.95.

7. A process as claimed in any preceding claim, wherein the organic solvent is selected from saturated aliphatic hydrocarbons, halogenated aliphatic hydrocarbons, alicyclic hydrocarbons, aromatic hydrocarbons, alkylated aromatics, halogenated aromatic compounds, aromatic ethers and aromatic ketones, or a mixture comprising two or more thereof.

8. A process as claimed in any preceding claim, wherein any low-boiling substances contained in the organic solvent phase are removed by distilling or evaporating prior to the separation of the organic solvent phase into trioxane and the organic solvent by distillation.

9. A process as claimed in any preceding claim, wherein the temperature of the synthesis reaction is in the range from 85 to 130°C.

## Patentansprüche

1. Verfahren zur Herstellung von Trioxan aus einer wäßrigen Lösung von Formaldehyd, dadurch gekennzeicnet, daß es die folgenden Stufen (1) bis (4):
(1) Herstellung einer wäßrigen Lösung von Formaldehyd einer hohen Konzentration, wobei die Formaldehyd-Konzentration der Lösung innerhalb eines Ausgangsbereichs von 40 bis 85 Gew.-% liegt;
(2) Überführung der wäßrigen Lösung von Formaldehyd einer hohen Konzentration, die in der obigen Stufe (1) erhalten wurde, in einen Reaktor, der mit einem festen Säure-Katalysator gepackt ist, um so Trioxan zu bilden;
(3) Einführen der wäßrigen Lösung von Formaldehyd, die das Trioxan enthält, das in der obigen Stufe (2) hergestellt wurde, in einen Extraktor ohne irgendeine Phasenänderung zu verursachen, um das Trioxan mit einem wasserunlöslichen, organischen Lösungsmittel zu extrahieren, das einen Siedepunkt hat, der höher ist als der des Trioxans, und das von irgendeiner azeotropen Zusammensetzung mit Trioxan frei ist; und
(4) Erhöhen der Formaldehyd-Konzentration der wäßrigen Phase - nachdem die Extraktion der obigen Stufe (3) vervollständigt ist - und Zurückführen der wäßrigen Phase zu dem Reaktor der Stufe (2), während die Phase des organisches Lösungsmittels in Trioxan und das organische Lösungsmittel durch Destillation abgetrennt werden, und Herausnehmen des Trioxan am Kopf einer Destillationssäule, und Zurückführen des erhaltenen, organischen Lösungsmittels vom Boden der Destillationssäule zu der Stufe (3),
umfaßt.

2. Verfahren gemäß Anspruch 1, worin der Ausgangsbereich der Formaldehyd-Konzentration der wäßrigen Lösung des für die Trioxan-Synthesereaktion zu verwendenden Formaldehyds 55 bis 80 Gew.-% beträgt.

3. Verfahren gemäß Anspruch 2, worin der Ausgangsbereich der Formaldehyd-Konzentration der wäßrigen Lösung des für die Trioxan-Synthesereaktion zu verwendenden Formaldehyds 60 bis 75 Gew.-% beträgt.

4. Verfahren gemäß irgendeinem der vorhergehenden Ansprüche, worin die wäßrige Lösung entweder durch Aufkonzentrieren - wenn die wäßrige Formaldehyd-Lösung eine Formaldehyd-Konzentration von weniger als 40 Gew.-% hat - der Lösung mittels Verdampfen oder Destillation zu der erwünschten Konzentration, oder durch Zugabe - wenn die wäßrige Formaldehyd-Lösung eine Formaldehyd-Konzentration von mehr als 85 Gew.-% hat, oder ein Formaldehydgas als das Ausgangsmaterial verwendet wird - von Wasser oder einer wäßrigen Formaldehyd-Lösung zu dem Ausgangsmaterial, um die erwünschte Konzentration zu ergeben, erhalten wird.

5. Verfahren gemäß irgendeinem der vorhergehenden Ansprüche, worin der feste Säure-Katalysator eine feste anorganische Säure, eine feste organische Säure oder eine Mischung derselben umfaßt.

6. Verfahren gemäß irgendeinem der vorhergehenden Ansprüche, worin das organische Lösungsmittel eine Dichte von 0,8 bis 0,95 hat.

7. Verfahren gemäß irgendeinem der vorhergehenden Ansprüche, worin das organische Lösungsmittel aus gesättigten aliphatischen Kohlenwasserstoffen, halogenierten aliphatischen Kohlenwasserstoffen, alicyclischen Kohlenwasserstoffen, aromatischen Kohlenwasserstoffen, alkylierten Aromaten, halogenierten aromatischen Verbindungen, aromatischen Ethern und aromatischen Ketonen oder einer Mischung, die zwei oder mehrere derselben umfaßt, ausgewählt ist.

8. Verfahren gemäß irgendeinem der vorhergehenden Ansprüche, worin alle niedrig-siedenden Substanzen, die in der Phase des organischen Lösungsmittels enthalten sind, durch Destillation oder Verdampfen vor der Abtrennung der Phase des organischen Lösungsmittels in Trioxan und des organischen Lösungsmittels durch Destillation entfernt werden.

9. Verfahren gemäß irgendeinem der vorhergehenden Ansprüche, worin die Temperatur der Synthesereaktion im Bereich von 85 bis 130 °C liegt.

## Revendications

1. Un procédé pour la production de trioxanne à partir d'une solution aqueuse de formaldéhyde, caractérisé en ce qu'il comprend les étapes (1) à (4) suivantes :
(1) préparer une solution aqueuse de formaldéhyde à haute concentration, la concentration en formaldéhyde de ladite solution étant comprise dans un intervalle de départ de 40 à 85 % en poids ;
(2) transférer la solution aqueuse de formaldéhyde à haute concentration obtenue dans l'étape (1) ci-dessus dans un réacteur garni d'un catalyseur acide solide, pour former ainsi du trioxanne ;
(3) introduire dans un extracteur la solution aqueuse de formaldéhyde contenant le trioxanne produit dans l'étape (2) ci-dessus, sans provoquer de changement de phase, pour extraire le trioxanne avec un solvant organique insoluble dans l'eau ayant un point d'ébullition supérieur à celui du trioxanne et ne formant aucune composition azéotrope avec le trioxanne ; et
(4) après l'achèvement de l'extraction de l'étape (3) ci-dessus, élever la concentration en formaldéhyde de la phase aqueuse et ramener la phase aqueuse dans le réacteur de l'étape (2), tout en séparant la phase au solvant organique en trioxanne et en solvant organique par distillation, et retirer le trioxanne du sommet d'une colonne de distillation et ramener à l'étape (3) le solvant organique obtenu au bas de la colonne de distillation.

2. Un procédé tel que revendiqué dans la revendication 1, dans lequel l'intervalle de départ de la concentration en formaldéhyde de la solution aqueuse de formaldéhyde à utiliser pour la réaction de synthèse du trioxanne est de 55 à 80 % en poids.

3. Un procédé tel que revendiqué dans la revendication 2, dans lequel l'intervalle de départ de la concentration en formaldéhyde de la solution aqueuse de formaldéhyde à utiliser pour la réaction de synthèse du trioxanne est de 60 à 75 % en poids.

4. Un procédé tel que revendiqué dans l'une quelconque des revendications précédentes, dans lequel ladite solution aqueuse est obtenue soit, lorsque la solution aqueuse de formaldéhyde a une concentration en formaldéhyde inférieure à 40 % en poids, en concentrant ladite solution par évaporation ou distillation jusqu'à la concentration souhaitée, soit, lorsque la solution aqueuse de formaldéhyde a une concentration en formaldéhyde dépassant 85 % en poids ou lorsque du formaldéhyde gazeux est utilisé comme matière d'alimentation, en ajoutant de l'eau ou une solution aqueuse de formaldéhyde à la matière d'alimentation pour obtenir la concentration souhaitée.

5. Un procédé tel que revendiqué dans l'une quelconque des revendications précédentes, dans lequel le catalyseur acide solide comprend un acide minéral solide, un acide organique solide ou un mélange d'entre eux.

6. Un procédé tel que revendiqué dans l'une quelconque des revendications précédentes, dans lequel le solvant organique a une densité de 0,8 à 0,95.

7. Un procédé tel que revendiqué dans l'une quelconque des revendications précédentes, dans lequel le solvant organique est choisi parmi les hydrocarbures aliphatiques saturés, les hydrocarbures aliphatiques halogénés, les hydrocarbures alicycliques, les hydrocarbures aromatiques, les composés aromatiques alkylés, les composés aromatiques halogénés, les éthers aromatiques et les cétones aromatiques, ou un mélange comprenant deux ou plusieurs d'entre eux.

8. Un procédé tel que revendiqué dans l'une quelconque des revendications précédentes, dans lequel toutes les substances de bas point d'ébullition éventuellement contenues dans la phase au solvant organique sont éliminées par distillation ou évaporation avant la séparation de la phase au solvant organique en trioxanne et en solvant organique par distillation.

9. Un procédé tel que revendiqué dans l'une quelconque des revendications précédentes, dans lequel la température de la réaction de synthèse se situe dans l'intervalle de 85 à 130°C.
